# EUROPÄISCHE PATENTSCHRIFT

(11) **EP 3 057 557 B1**
(45) Veröffentlichungstag und Bekanntmachung des Hinweises auf die Patenterteilung: **21.03.2018**
(21) Anmeldenummer: 14796722.8
(22) Anmeldetag: 17.10.2014
(51) Int. Cl.: A61G 13/12, A61G 13/10

(54) **RÖNTGENOPTIMIERTE VORRICHTUNG ZUM LAGERN EINES PATIENTEN**
X-RAY-OPTIMIZED DEVICE FOR SUPPORTING A PATIENT
DISPOSITIF OPTIMISÉ POUR LA RADIOGRAPHIE POUR POSITIONNER UN PATIENT

(30) Priorität: 18.10.2013 DE 102013111523
(43) Veröffentlichungstag der Anmeldung: 24.08.2016
(73) Patentinhaber: Maquet GmbH, 76437 Rastatt (DE)
(72) Erfinder: KAISER, Jochen, 76185 Karlsruhe (DE); WYSLUCHA, Ulrich, 76356 Weingarten (DE); HUND, Siegfried, 77704 Oberkirch (DE)
(74) Vertreter: Zacco GmbH
(86) Internationale Anmeldenummer: PCT/EP2014/072323
(87) Internationale Veröffentlichungsnummer: WO 2015/055818

(56) Entgegenhaltungen:
- US-A- 5 088 706
- US-A1- 2006 255 220

## Beschreibung

Die Erfindung betrifft eine Vorrichtung zum Lagern eines Patienten, die eine an einem Operationstisch befestigbare erste Schiene, eine ebenfalls an dem Operationstisch befestigbare zweite Schiene sowie eine Verbindungseinheit umfasst, die das dem Operationstisch abgewandte erste Ende der ersten Schiene mit dem dem Operationstisch abgewandten ersten Ende der zweiten Schiene verbindet, so dass die beiden Schienen in einem vorbestimmten Abstand zueinander angeordnet sind. Ferner weist die Vorrichtung eine an der ersten Schiene angeordnete erste Patientenlagereinheit zum Lagern des Patienten und eine an der zweiten Schiene angeordnete zweite Patientenlagereinheit zum Lagern des Patienten auf.

Bei manchen Operationen, insbesondere bei Operationen an der Wirbelsäule, ist es notwendig, dass der Patient während der Operation geröntgt werden kann. Hierzu werden häufig C-förmige Röntgenapparate verwendet, wobei in der Öffnung des "C" der Patient gelagert ist. Insbesondere werden auch um bis zu 270° verschwenkbare C-Bögen zur Aufnahme von 3D-Bildern verwendet. Die Verwendung von klassischen Operationstischen zur Lagerung des Patienten ist nicht oder nur eingeschränkt möglich, da ein Röntgen nur in sehr beschränktem Umfang möglich ist. Zur Aufnahme von 3D-Aufnahmen muss der C-Bogen möglichst nah am Patienten über einen möglichst großen Bereich bewegt werden können. Die standardmäßigen Patientenauflegen von Operationstischen sind hierfür zu breit ausgebildet. Darüber hinaus umfasst der Operationstisch häufig dicke, metallhaltige Konstruktionen, so dass ein Röntgen nur in unzureichender Qualität möglich ist. Erschwerend kommt hinzu, dass die Dicke und Konturen der Konstruktionen sehr unterschiedlich sind, was die Qualität des Röntgenbildes weiter negativ beeinflusst und die Röntgenaufnahme über den C-Bogen limitiert.
Daher werden Vorrichtungen zum Lagern des Patienten verwendet, die an den Operationstisch angebaut werden können. Der Patient liegt dann mit dem Oberkörper, an dem operiert wird und der geröntgt werden muss, auf der angebauten Vorrichtung und lediglich mit seinen Beinen auf dem eigentlichen Operationstisch.
Eine solche Vorrichtung zum Lagern eines Patienten während einer Operation ist beispielsweise aus dem Dokument US 7,600,281 B2 bekannt. Die dort beschriebene Vorrichtung umfasst zwei parallel zueinander verlaufende Holme, die an der einen Seite am Operationstisch und an der anderen Seite an einem Ständer befestigt sind. An den Holmen sind mehrere Auflageflächen vorgesehen, auf denen der Patient, insbesondere der Oberkörper und die Hüften gelagert werden können. Diese Auflageflächen ragen hierbei über den gesamten Bereich zwischen den beiden Holmen.
Die zuvor beschriebene Vorrichtung hat den Nachteil, dass durch die Auflageelemente die Qualität eines aufgenommenen Röntgenbildes negativ beeinflusst werden kann. Ferner ist die Aufnahme von 3D-Bildern nicht oder nur sehr beschränkt möglich, da die Bauteile einer Bewegung des C-Bogens im Wege stehen. Die Konturen der Auflageelemente finden sich im Röntgenbild wieder, was ggf. zu Fehlinterpretationen führen kann. Darüber hinaus ermöglichen die starren Auflageelemente nur eine unzureichende Anpassung an die individuelle Anatomie des Patienten, so dass dieser ggf. nicht optimal für die Operation gelagert werden kann. Eine weitere Vorrichtung zum Lagern eines zu röntgenden Patienten während einer Operation ist aus US 2006/255220 bekannt. Es ist Aufgabe der Erfindung, eine Vorrichtung zum Lagern eines Patienten anzugeben, die eine optimale Lagerung des Patienten und dennoch die Aufnahme qualitativ hochwertiger Röntgenaufnahmen, insbesondere auch in 3D, ermöglicht.

Diese Aufgabe wird durch eine Vorrichtung mit den Merkmalen des Anspruchs 1 gelöst. Vorteilhafte Weiterbildungen der Erfindung sind in den abhängigen Ansprüchen angegeben.

Erfindungsgemäß ist die erste Patientenlagereinheit ausschließlich an der ersten Schiene und die zweite Patientenlagereinheit ausschließlich an der zweiten Schiene gelagert. Die erste und zweite Patientenlagereinheit sind derart gelagert und ausgebildet, dass auch bei gegenüberliegender Anordnung der Patientenlagereinheit zwischen den beiden Patientenlagereinheiten ein materialfreier Röntgenbereich ausgebildet ist. Hierdurch wird erreicht, dass in dem Röntgenbereich sich kein Röntgenstrahlen absorbierendes oder reflektierendes Material befindet und somit eine komplette Durchleuchtbarkeit eines auf den Patientenlagereinheiten gelagerten Patienten gegeben ist. Somit wird ein völlig unverfälschtes Röntgenbild des Patienten, insbesondere auch in 3D, zumindest in dem materialfreien Bereich, der auch als Röntgenbereich bezeichnet wird, ermöglicht. Ferner ergibt sich ein schlanker Aufbau, wodurch der C-Bogen optimal bewegt werden kann. Darüber hinaus ermöglicht der Abstand zwischen den Patientenlagereinheiten, dass der Bauch des Patienten nicht aufliegt, was insbesondere bei untersetzten Patienten eine bessere Lagerung ermöglicht.

Unter einem materialfreien Bereich wird insbesondere verstanden, dass die beiden Patientenlagereinheiten in einem vorbestimmten Abstand zueinander angeordnet sind und sich zwischen den Patientenlagereinheiten ein Raum ergibt, in dem sich kein weiteres Bauteil befindet, wenn kein Röntgenapparat installiert ist.

Unter der gegenüberliegenden Anordnung wird insbesondere verstanden, dass beide Patientenlagereinheiten den gleichen Abstand zur ersten Verbindungseinheit haben. Zwischen der ersten und der zweiten Patientenlagereinheit besteht insbesondere keine Verbindung, d.h. dass insbesondere kein die erste oder die zweite Patientenlagereinheit verbindendes Element vorgesehen ist. Dies hat zum einen den Vorteil, dass der materialfreie Röntgenbereich verwirklicht wird, und zum anderen, dass die beiden Patientenlagereinheiten unabhängig voneinander bewegt, insbesondere auf den Schienen verschoben, werden können.

Der materialfreie Röntgenbereich ist insbesondere auch dann ausgebildet, wenn die beiden Patientenlagereinheiten parallel nebeneinander angeordnet sind, d.h. wenn die beiden Patientenlagereinheiten im gleichen Abstand zu der ersten Verbindungseinheit auf den Schienen angeordnet sind.

Es ist vorteilhaft, wenn das dem ersten Ende der ersten Schiene entgegengesetzte zweite Ende der ersten Schiene und das dem ersten Ende der zweiten Schiene entgegengesetzte zweite Ende der zweiten Schiene über eine zweite Verbindungseinheit miteinander verbunden sind. Somit wird sichergestellt, dass die beiden Schienen einen vorbestimmten Abstand zueinander haben, so dass zur Verwendung der bestimmungsgemäßen Patientenlagereinheiten die Größe dieser Patientenlagereinheiten derart auf den Abstand abgestimmt sind, dass ein ausreichend großer Freiraum zwischen den Patientenlagereinheiten und somit ein ausreichend großer materialfreier Röntgenbereich gegeben ist. Die Schienen sind insbesondere aus einem durchleuchtbaren Material, beispielsweise CFR, so dass diese bei einer Schrägbewegung des C-Bogens bei der Aufnahme von 3D-Bildern durchleuchtet werden können und sich möglichst wenig Material im Strahlengang befindet.

Die zweite Verbindungseinheit ist insbesondere in Form einer Stange ausgebildet, die an den zweiten Enden der Schienen befestigt ist und somit diese in einem Abstand zueinander hält.

Die erste und die zweite Schiene sind insbesondere parallel zueinander angeordnet, so dass diese überall den gleichen Abstand zueinander haben. Dadurch haben auch die auf ihnen gelagerten Patientenlagereinheiten unabhängig von ihrer Position auf den Schienen den gleichen Abstand zur jeweils anderen Schiene. Auch hierdurch wird sichergestellt, dass ein ausreichend großer Röntgenbereich gegeben ist.

Die erste Schiene und die zweite Schiene sind vorzugsweise ausschließlich über die erste Verbindungseinheit und die zweite Verbindungseinheit miteinander verbunden. Dies hat den Vorteil, dass ein möglichst großer materialfreier Bereich zum Röntgen gegeben ist, da, egal an welcher Stelle auf den Schienen die Patientenlagereinheiten angeordnet sind und somit der Patient gelagert ist, jeweils ein Freiraum zwischen den Patientenlagereinheiten gegeben ist, in dem keine Verbindungselemente zwischen den Schienen die Durchgängigkeit der Röntgenstrahlen beeinträchtigen.

Die erste Verbindungseinheit ist insbesondere Teil eines Ständers, über den die Schienen an einer Seite auf dem Boden gelagert werden können. Dieser Ständer ist vorzugsweise derart ausgebildet, dass er höhenverstellbar ist, so dass die Höhe der Schienen relativ zum Boden verändert werden kann und somit an die Höhe des Operationstisches angepasst werden kann. Ferner kann die Höhe ergonomisch günstig für die operierenden Ärzte eingestellt werden.

Die anderen Enden der Schienen sind insbesondere an dem Operationstisch befestigt, der ebenfalls höhenverstellbar ist, so dass zusammen mit dem höhenverstellbaren Ständer die Schienen derart verstellt werden können, dass sie horizontal ausgerichtet bleiben.

Bei einer besonderes bevorzugten Ausführungsform der Erfindung ist an dem dem ersten Ende entgegengesetzten zweiten Ende der ersten Schiene eine erste Befestigungseinheit zur Befestigung der Vorrichtung an dem Operationstisch und an dem dem ersten Ende entgegengesetzten zweiten Ende der zweiten Schiene eine zweite Befestigungseinheit zur Befestigung der Vorrichtung an dem Operationstisch angeordnet. Diese Befestigungseinheiten sind insbesondere derart ausgebildet, dass die Schienen auf einfache Weise reversibel an dem Operationstisch sicher befestigt werden können, aber dennoch ein unbeabsichtigtes Lösen vermieden wird.

Weiterhin ist es vorteilhaft, wenn an der ersten Schiene eine dritte Patientenlagereinheit zur Lagerung des Patienten und/oder an der zweiten Schiene eine vierte Patientenlagereinheit zur Lagerung des Patienten angeordnet ist. Auch hierbei ist es wiederum so, dass die dritte Patientenlagereinheit ausschließlich an der ersten Schiene und die vierte Patientenlagereinheit ausschließlich an der zweiten Schiene befestigt ist. Ferner sind auch diese Patientenlagereinheiten derart ausgebildet, dass unabhängig von ihrer Anordnung auf den Schienen immer ein materialfreier Röntgenbereich ausgebildet ist.

Die erste Patientenlagereinheit und/oder die dritte Patientenlagereinheit sind insbesondere auf der ersten Schiene in Längsrichtung der ersten Schiene unabhängig voneinander verschiebbar. Ebenso sind vorzugsweise die zweite Patientenlagereinheit und die vierte Patientenlagereinheit auf der zweiten Schiene in Längsrichtung der zweiten Schiene unabhängig voneinander verschiebbar gelagert. Insbesondere lassen sich auch die erste und die zweite Patientenlagereinheit sowie die dritte und die vierte Patientenlagereinheit unabhängig voneinander verschieben, da zwischen ihnen keine Verbindungselemente bestehen. Somit kann die Anordnung der vier Patientenlagereinheiten optimal an die individuelle Anatomie des Patienten angepasst werden. Insbesondere müssen nicht die an den beiden Schienen nebeneinander angeordneten Patientenlagereinheiten exakt parallel zueinander angeordnet sein, sondern können auch ein wenig versetzt angeordnet werden.
Die erste Patientenlagereinheit und die zweite Patientenlagereinheit dienen insbesondere zur Lagerung des Oberkörpers des Patienten, wobei die dritte und die vierte Patientenlagereinheit vorzugsweise zur Lagerung der Hüften des Patienten vorgesehen sind. Anders als bei herkömmlichen Vorrichtungen können durch die individuelle Verschiebbarkeit der vier Patientenlagereinheiten somit beispielsweise die beiden zur Lagerung der Hüften dienenden Patientenlagereinheiten in einem unterschiedlichen Abstand zum Operationstisch angeordnet werden. Gleiches gilt auch für die zur Lagerung des Oberkörpers vorgesehenen Patientenlagereinheiten.
Ferner ist es vorteilhaft, wenn die erste, zweite, dritte und/oder vierte Patientenlagereinheit an der jeweiligen Schiene über eine lös- und wiederherstellbare Schnellbefestigung befestigbar sind. Somit können diese vor der Operation einfach angebracht bzw. nach der Operation einfach gelöst werden. Insbesondere können die Patientenlagereinheiten auch bei bereits befestigten Schienen angebracht werden, da über die Schnellverschlusseinrichtungen diese in radialer Richtung auf die Schiene aufgebracht werden können und nicht axial auf die Schienen aufgesteckt werden müssen. Die Schnellverschlussvorrichtung ist derart ausgebildet, dass eine Befestigungseinheit der Patientenlagereinheit einen U-förmigen Grundkörper umfasst, der von oben auf die rechteckigen Schienen aufgesetzt wird. Am Ende einer der beiden Flanken des U-förmigen Grundkörpers ist ein Riegel verschwenkbar angeordnet, der über eine Schraube an dem Ende der anderen Flanke des U-förmigen Grundkörpers mit Hilfe einer manuelle ohne weitere Hilfsmittel betätigbare Schraube oder Mutter angeschraubt werden kann, so dass eine geschlossene Kontur gebildet ist, die ein sicheres Befestigen der Patientenauflageeinheiten an den Schienen ermöglicht und somit ein unbeabsichtigtes Lösen während der Operation verhindert. Im U-förmigen Grundkörper können Antirutschpads vorgesehen sein, die ein Verschieben der Patientenlagereinheit auf der Schiene verhindern, wenn der Riegel geschlossen ist.

Die erste, zweite, dritte oder vierte Patientenlagereinheit umfasst vorzugsweise jeweils eine Befestigungseinheit zur Befestigung an der Schiene, ein Auflagepad, auf dem ein Polster angeordnet sein kann, auf dem der Patient aufliegt, und eine Höhenverstelleinheit. Die Höhenverstelleinheit ist zwischen der Befestigungseinheit und dem Auflagepad angeordnet und dient dazu, den Abstand zwischen dem Auflagepad und den Befestigungseinheiten variieren zu können. Somit kann auch der Abstand zwischen dem Auflagepad und der Schiene verändert werden, so dass auch in vertikale Richtung eine Anpassung der Lagerung des Patienten an die individuelle Anatomie des Patienten vorgenommen werden kann. Insbesondere die Kombination aus dem unabhängigen Verschieben der Patientenlagereinheit auf den Schienen mit der Höhenverstellbarkeit der Auflagepads bietet eine optimale Anpassung an den jeweiligen Patienten.

Die Auflagepads sind insbesondere fest mit der Höhenverstelleinheit verbunden und können quer zur Schiene in Richtung der anderen Schienen verstellt werden, so dass der Abstand zwischen den Auflagepads benachbarter Patientenlagereinheiten eingestellt werden kann. Die Auflagepads umfassen insbesondere eine an der Höhenverstelleinheit befestigte Tragestruktur und ein auf dieser angeordnetes Polster.

Die Auflagepads der Patientenlagereinheiten sind insbesondere aus einem für ein qualitativ hochwertiges Röntgenbild ausreichend röntgenstrahlendurchlässigen Material ausgebildet. Darüber hinaus sind die Auflagepads vorzugsweise derart geformt, dass sie eine homogene Kontur, insbesondere eine gleichbleibende Dicke und Materialstärke, aufweisen. Diese Homogenität und das röntgenstrahlendurchlässige Material ermöglichen, dass der materialfreie Röntgenbereich durch einen an diesen angrenzenden Zusatzröntgenbereich vergrößert werden kann. Dieser Zusatzröntgenbereich, also der Bereich, in dem der Patient auf den Auflagepads aufliegt, aber noch nicht im Bereich der Schienen bzw. Höhenverstelleinheit angeordnet ist, bietet zwar nicht ein so optimales Röntgenbild wie der materialfreie Röntgenbereich zwischen den Auflagepads, aber durch die spezielle Ausbildung der Auflagepads immer noch ein sehr gutes Röntgenbild. Somit kann nahezu der gesamte Bereich zwischen den beiden Schienen zur Erzielung eines qualitativ hochwertigen Röntgenbildes verwendet werden. Der materialfreie Röntgenbereich und der Zusatzröntgenbereich bilden zusammen einen erweiterten Röntgenbereich.

Die Auflagepads sind insbesondere aus einem Kunststoff ausgebildet, so dass zum einen eine gute Durchlässigkeit von Röntgenstrahlen erreicht wird und zum anderen durch die hohe Festigkeit eines kohlefaserverstärkten Kunststoffes die Auflagepads möglichst dünn ausgebildet werden können, so dass sie die Röntgenstrahlen möglichst wenig beeinflussen.

Bei einer besonders bevorzugten Ausführungsform sind sowohl die erste Schiene, die zweite Schiene, die erste Patientenlagereinheit, die zweite Patientenlagereinheit, die dritte Patientenlagereinheit und/oder die vierte Patientenlagereinheit vollständig metallfrei ausgebildet. Dies hat den Vorteil, dass, wenn ein Röntgenbild außerhalb des erweiterten Röntgenbereiches aufgenommen werden muss, dies immer noch mit einer annehmbaren Qualität möglich ist. Die zuvor genannten Einheiten können insbesondere aus einem kohlefaserverstärkten Kunststoff oder einem anderen röntgenstrahlendurchlässigen Material ausgebildet sein. Hierdurch wird erreicht, dass bei der Aufnahme von 3D-Aufnahmen auch beim Durchleuchten der Bauteile, insbesondere der Schienen, qualitativ gute Röntgenaufnahmen möglich sind.

Die Breite der Befestigungseinheiten der Patientenlagereinheiten und die Breite der Höhenverstelleinheiten der Patientenlagereinheiten entsprechen jeweils in etwa der Breite der jeweiligen Schiene bzw. sind nur geringfügig breiter, so dass ein möglichst großer erweiterter Röntgenbereich ausgebildet ist, der nahezu den gesamten Bereich zwischen den beiden Schienen umfasst.
Ferner ist es vorteilhaft, wenn die erste Schiene und die zweite Schiene baugleich sind, so dass nur eine Art von Schienen gefertigt werden muss. Ebenso ist es vorteilhaft, wenn die erste, die zweite, die dritte und/oder die vierte Patientenlagereinheit ebenfalls baugleich sind. Alternativ können auch nur die Befestigungseinheiten und die Höhenverstelleinheiten gleich sein und die Auflagepads an die bestimmungsgemäßen Körperregionen angepasst sein.

Ein weiterer Aspekt betrifft eine Anordnung, die einen Operationstisch und eine Vorrichtung zur Lagerung eines Patienten gemäß der zuvor beschriebenen Art umfasst. Die Vorrichtung zum Lagern des Patienten ist hierbei an dem Operationstisch befestigt. Die Vorrichtung zum Lagern des Patienten kann insbesondere gemäß einem der Ansprüche 1 bis 13 ausgebildet sein.

Ein weiterer Aspekt betrifft eine Anordnung, die einen Operationstisch, eine an dem Operationstisch befestigte Vorrichtung zum Lagern eines Patienten gemäß der zuvor beschriebenen Art sowie einen Röntgenapparat zum Röntgen eines auf der Vorrichtung zum Lagern des Patienten gelagerten Patienten umfasst. Auch hier kann die Vorrichtung zum Lagern des Patienten insbesondere gemäß den Merkmalen eines der Ansprüche 1 bis 13 ausgebildet sein. Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Fig.1: eine schematische, perspektivische Darstellung einer Anordnung zum Lagern eines zu röntgenden Patienten während einer Operation;
- Fig.2: eine Draufsicht auf eine Vorrichtung nach Fig.1; und
- Fig.3: eine Seitenansicht zweier Patientenlagereinheiten der Vorrichtung nach den Fig.1 und 2.

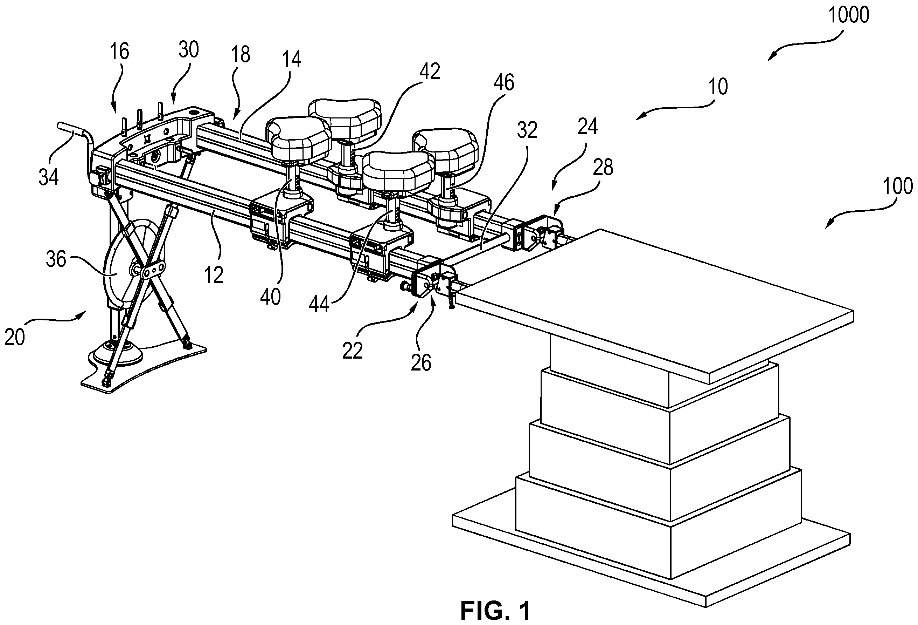

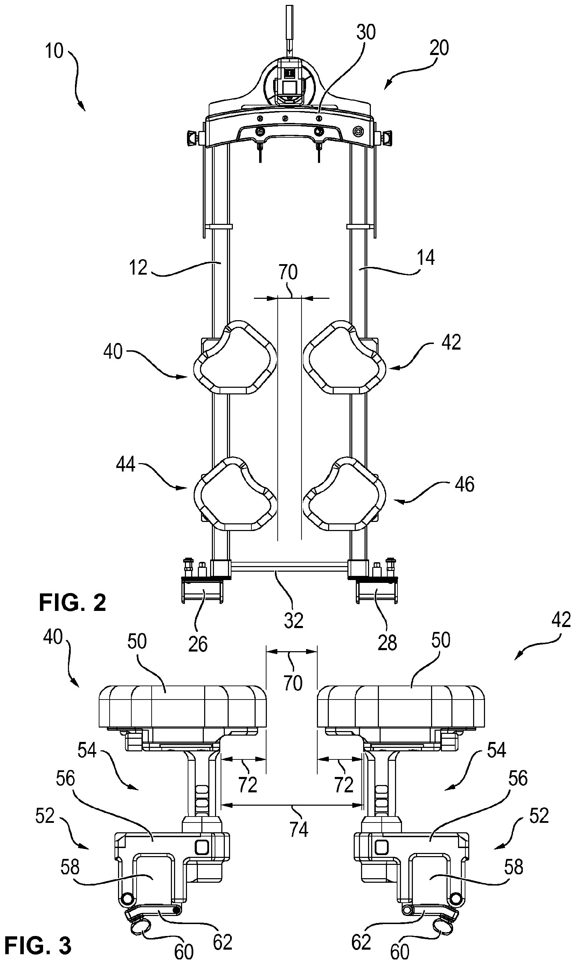

In Fig.1 ist eine schematische, perspektivisch Darstellung einer Anordnung 1000 zum Lagern eines Patienten während einer Operation dargestellt. Die Anordnung umfasst einen Operationstisch 100 sowie eine Vorrichtung 10 zum Lagern des zu röntgenden Körperbereichs des Patienten. Fig.2 zeigt eine Draufsicht auf diese Vorrichtung 10 nach Fig.1.

Die Vorrichtung 10 wird insbesondere verwendet, um Patienten während einer Rückenoperation zu lagern. Bei solchen Rückenoperationen, insbesondere der Wirbelsäule, müssen die Patienten in der Regel während der Operation geröntgt werden, wozu ein C-förmiges Röntgengerät verwendet wird. Insbesondere wird der C-Bogen des Röntgengeräts zur Aufnahme von 3D-Bildern um den Patienten bewegt. Wie im Folgenden noch näher ausgeführt wird, ist die Vorrichtung 10 derart ausgebildet, dass mit ihr auf einfache Weise ein qualitativ hochwertiges Röntgenbild erzeugt werden kann. Ein Patient kann nicht auf einem "normalen" Operationstisch 100 gelagert werden, da ein solcher Operationstisch ein Röntgen mit der notwendigen Qualität und in den erforderlichen Bereichen nicht ermöglicht. Zum einen begrenzt die massive Fußsäule des Operationstisches 100 die mögliche Röntgenlänge und zum anderen umfasst ein normaler Operationstisch 100 zu viele metallhaltige konstruktive Elemente, die das Röntgenbild negativ beeinflussen. Außerdem ist die Patientenlagerfläche des Operationstisches 100 zu breit für die Aufnahme von 3D-Aufnahmen mit Hilfe eines verschwenkbaren C-Bogens.

Die Vorrichtung 10 umfasst zwei Schienen 12, 14, deren erste Enden 16, 18 an einem Ständer 20 gelagert sind. Die den ersten Enden 16, 18 entgegengesetzten zweiten Enden 22, 24 der Schienen 12, 14 können über Befestigungseinheiten 26, 28 an dem Operationstisch 100, insbesondere an Schnittstellen zur Verbindung des Operationstisches 100 mit Beinplatten, befestigt werden.

Der Ständer 20 dient zum einen zum Lagern der Schienen 12, 14 auf dem Boden und zum anderen dazu, um eine vorbestimmten Abstand zwischen den Schienen 12, 14 sicherzustellen, indem der Ständer 20 eine Verbindungseinheit 30 umfasst, über die die beiden ersten Enden 16, 18 der Schienen 12, 14 miteinander verbunden sind.

Ebenso sind die zweiten Enden 22, 24 der Schienen 12, 14 ebenfalls über eine Verbindungseinheit 32 miteinander verbunden, so dass der gewünschte Abstand zwischen den Schienen 12, 14 eingehalten ist. Die Schienen 12, 14 verlaufen parallel zueinander.

Der Ständer 20 ist derart ausgebildet, dass er höhenverstellbar ist, d.h. dass der Abstand der Schienen 12, 14 zum Boden verändert werden kann. Hierzu umfasst der Ständer 20 zum einen eine Handkurbel 34 mit deren Hilfe die Höhe verändert werden kann und zum anderen ein Handrad 36 zum Feststellen und Versteifen des Standfußes des Ständers 20.

Der Operationstisch, an dem die Schienen 12, 14 über die Befestigungseinheiten 26, 28 befestigt sind, ist vorzugsweise mit Hilfe eines Stellantriebs höhenverstellbar, so dass durch entsprechendes Einstellen des Ständers 20 und des Operationstisches die Schienen 12, 14 in einer geeigneten Höhe horizontal angeordnet werden können.

Auf den Schienen 12, 14 sind jeweils zwei Patientenlagereinheiten 40 bis 46 zum Lagern des Patienten angeordnet. In Fig.3 sind zwei dieser Patientenlagereinheiten 40, 42 in einer Seitenansicht dargestellt, wobei zur Vereinfachung der Darstellung die Schienen 12, 14 nicht dargestellt wurden.

Die Patientenlagereinheiten 40 bis 46 umfassen jeweils ein Auflagepad 50, auf dem der Patient aufliegt. Ferner haben die Patientenlagereinheiten 40 bis 46 jeweils eine Befestigungseinheit 52 zur Befestigung der jeweiligen Patientenlagereinheit 40 bis 46 an der jeweiligen Schiene 12, 14 sowie eine Höhenverstelleinheit 54, über die das Auflagepad 50 mit der Befestigungseinheit 52 verbunden ist und über die der Abstand zwischen dem Auflagepad 50 und der Befestigungseinheit 52 eingestellt werden kann.

Die Befestigungseinheit 52 umfasst einen U-förmigen Grundkörper 56, in dessen Aussparung 58 die jeweilige Schiene 12, 14 aufgenommen werden kann. An dem offenen Ende des U-förmigen Grundkörpers ist ein über eine Schraube 60 verriegelbarer Riegel 62 vorgesehen. Zur Montage der Patientenlagereinheit 40 bis 46 wird die Befestigungseinheit 52 beim geöffneten Riegel 62 auf die jeweilige Schiene 12, 14 aufgesetzt, so dass diese in der U-förmigen Aussparung 58 aufgenommen ist. Anschließend wird der Riegel 62 verschlossen und über die Schraube 60 befestigt, so dass eine sichere und dennoch einfache und schnelle Befestigung der Patientenlagereinheit 40 bis 46 an den Schienen 12, 14 möglich ist.

Die Patientenlagereinheiten 40, 42 dienen insbesondere zur Lagerung des Oberkörpers eines Patienten, wohingegen die Patientenlagereinheiten 44, 46 zur Lagerung der Hüfte des Patienten dienen. Der Patient liegt somit mit seinem Kopf in Richtung des Ständers 20, wobei seine Beine auf einem Teil der Patientenlagerfläche des eigentlichen Operationstisches 100 aufliegen. Zur Stützung des Kopfes sind insbesondere noch weitere, nicht dargestellte Patientenlagereinheiten an den Schienen 12, 14 befestigt.

Die Patientenlagereinheiten 40, 44 sind ausschließlich an der ersten Schiene 12, die Patientenlagereinheiten 42, 46 ausschließlich an der zweiten Schiene 14 befestigt. Es bestehen keine Verbindung zwischen den nebeneinander angeordneten Patientenlagereinheiten 40 und 42 sowie 44 und 46.

Die Patientenlagereinheiten 40 bis 46 sind daher unabhängig voneinander einzeln auf den Schienen 12, 14 verschiebbar, so dass zusammen mit der Höhenverstellung der Patientenlagereinheiten 40 bis 46 eine optimale Anpassung an die individuelle Anatomie eines zu operierenden Patienten möglich ist. Insbesondere müssen somit die Patientenlagereinheiten 40 bis 46, anders als dargestellt, nicht immer direkt nebeneinander angeordnet sein. Darüber hinaus können die Auflagepads 50 in unterschiedlichen Höhen angeordnet sein. Ferner kann der Abstand zwischen den Auflagepads 50 von benachbarten Patientenlagereinheiten 40 und 42 bzw. 44 und 46 variiert werden, indem die Auflagepads auf der Höhenverstelleinheit 54 entsprechend quer verschieblich gelagert sind.

Wie den Fig.2 und 3 gut zu entnehmen ist, sind der Abstand zwischen den Schienen 12 und 14 und die Abmessungen der Patientenlagereinheiten 40 bis 46 derart gewählt, dass zwischen den einander zugewandten Seiten an verschiedenen Schienen 12, 14 befestigter Auflagepads 50 ein Freiraum ausgebildet ist. Über diesen Abstand der Auflagepads 50 sowie durch das Nichtverbinden der nebeneinander angeordneten Patientenauflageeinheiten 40, 42 bzw. 44, 46 wird erreicht, dass ein materialfreier Röntgenbereich ausgebildet ist, der in Fig.2 durch ein Rechteck angedeutet und mit dem Bezugszeichen 70 bezeichnet ist. In diesem materialfreien Röntgenbereich 70 werden die Röntgenstrahlen von keinem Material beeinflusst, so dass ein optimales Röntgenbild, insbesondere der bei korrekter Lagerung des Patienten in diesem Bereich angeordneten Wirbelsäule, möglich ist.

Die Auflagepads 50 der Patientenlagereinheiten 40 bis 46 sind insbesondere aus einem röntgenstrahlendurchlässigen Material, beispielsweise einem kohlefaserverstärkten Kunststoff, ausgebildet. Darüber hinaus ist ihre Form in einem an den Röntgenbereich angrenzenden Bereich möglichst homogen und dünn ausgebildet, um Röntgenstrahlenabsorptionsunterschiede, die sich in einem Röntgenbild abbilden würden, zu vermeiden. Somit sind die Auflagepads 50, zumindest in den Bereichen, in denen sie von der Befestigungseinheit 52 und der Höhenverstelleinheit 54 in Richtung des Röntgenbereichs 50 überstehen, für Röntgenstrahlen sehr gut durchlässig, so dass sich, wie in Fig.3 gezeigt, jeweils ein zusätzlicher Röntgenbereich 72 ergibt, in dem ein Röntgenbild mit einer sehr guten Qualität aufgenommen werden kann. Zusammen mit dem Röntgenbereich 70 ergibt sich somit ein erweiterter Röntgenbereich 74, der während der Operation genutzt werden kann.

Die Befestigungseinheit 52 und die Höhenverstelleinheit 54 sind derart ausgebildet, dass sie nur möglichst wenig über die Schiene 12, 14 in Richtung des Röntgenbereiches 70 überstehen, so dass ein möglichst großer Zusatzröntgenbereich 72 ausgebildet ist und somit der erweiterte Röntgenbereich 74 möglichst groß ist.

Die zuvor beschriebene Vorrichtung 10 ermöglicht somit zum einen eine möglichst große Flexibilität bei der Anpassung an die individuelle Anatomie eines Patienten und zum anderen die Aufnahme möglichst hochqualitativer Röntgenbilder.

### Bezugszeichenliste

- 10: Vorrichtung
- 12, 14: Schiene
- 16, 18, 22, 24: Ende
- 20: Ständer
- 30, 32: Verbindungseinheit
- 34: Kurbel
- 36: Handrad
- 40, 42, 44, 46: Patientenlagereinheit
- 50: Auflagepad
- 52: Befestigungseinheit
- 54: Höhenverstelleinheit
- 56: Grundkörper
- 58: Aussparung
- 60: Schraube
- 62: Riegel
- 70: Röntgenbereich
- 72: Zusatzröntgenbereich
- 74: erweiterter Röntgenbereich
- 100: Operationstisch
- 1000: Anordnung

## Patentansprüche

1. Vorrichtung zum Lagern eines zu röntgenden Patienten während einer Operation,
mit einer an einem Operationstisch befestigbaren ersten Schiene (12),
einer an dem Operationstisch befestigbaren zweiten Schiene (14),
mindestens einer ersten Verbindungseinheit (30), die das dem Operationstisch abgewandte erste Ende (16) der ersten Schiene (12) mit dem dem Operationstisch abgewandten ersten Ende (18) der zweiten Schiene (14) verbindet, so dass die beiden Schienen (12, 14) in einem vorbestimmten Abstand zueinander angeordnet sind,
einer an der ersten Schiene (12) angeordneten ersten Patientenlagereinheit (40) zur Auflage eines Körperbereichs des Patienten, und
mit einer an der zweiten Schiene (14) angeordneten zweiten Patientenlagereinheit (42) zur Auflage eines Körperbereichs des Patienten,
wobei die erste Patientenlagereinheit (40) ausschließlich an der ersten Schiene (12) und die zweite Patientenlagereinheit (42) ausschließlich an der zweiten Schiene (14) gelagert und derart ausgebildet sind, dass auch bei einer gegenüberliegenden Anordnung der Patientenlagereinheiten zwischen den beiden Patientenlagereinheiten (40, 42) ein materialfreier Röntgenbereich (70) ausgebildet ist,
**dadurch gekennzeichnet, dass** die erste und die zweite Patientenlagereinheit (40, 42) jeweils eine Befestigungseinheit aufweisen, die einen U-förmigen Grundkörper umfasst, der von oben auf die rechteckigen Schienen (12, 14) aufsetzbar ist, und dass am Ende einer der beiden Flanken des U-förmigen Grundkörpers ein Riegel (60) verschwenkbar angeordnet ist, der über eine Schraube (60) an dem Ende der anderen Flanke des U-förmigen Grundkörpers mit Hilfe einer manuell ohne weitere Hilfsmittel betätigbaren Schraube (60) oder Mutter anschraubbar ist.

2. Vorrichtung (10) nach Anspruch 1, **dadurch gekennzeichnet, dass** das dem ersten Ende (16) entgegengesetzte zweite Ende (22) der ersten Schiene (12) und das dem ersten Ende (18) entgegengesetzte zweite Ende (24) der zweiten Schiene (14) über eine zweite Verbindungseinheit (32) miteinander verbunden sind.

3. Vorrichtung (10) nach Anspruch 1, **dadurch gekennzeichnet, dass** die erste Schiene (12) und die zweite Schiene (14) ausschließlich über die erste Verbindungseinheit (30) verbunden sind.

4. Vorrichtung (10) nach einem der vorhergehenden Ansprüche, **dadurch gekennzeichnet, dass** die erste Verbindungseinheit (30) von einem Ständer (20), insbesondere einem höhenverstellbaren Ständer (20), gestützt ist.

5. Vorrichtung (10) nach einem der vorhergehenden Ansprüche, **dadurch gekennzeichnet, dass** an dem dem ersten Ende (16) entgegengesetzten zweiten Ende (22) der ersten Schiene (12) eine erste Befestigungseinheit (26) zur Befestigung der Vorrichtung (10) an dem Operationstisch und an dem dem ersten Ende (18) entgegengesetzten zweiten Ende (24) der zweiten Schiene (14) eine zweite Befestigungseinheit (28) zur Befestigung der Vorrichtung (10) an dem Operationstisch angeordnet sind.

6. Vorrichtung (10) nach einem der vorhergehenden Ansprüche, **dadurch gekennzeichnet, dass** an der ersten Schiene (12) eine dritte Patientenlagereinheit (44) zur Lagerung des Patienten und/oder an der zweiten Schiene (14) eine vierte Patientenlagereinheit (46) zur Lagerung des Patienten angeordnet sind.

7. Vorrichtung (10) nach Anspruch 6, **dadurch gekennzeichnet, dass** die erste Patientenlagereinheit (40) und/oder die dritte Patientenlagereinheit (44) auf der ersten Schiene (12) und/oder die zweite Patientenlagereinheit (42) und/oder die vierte Patientenlagereinheit (46) auf der zweiten Schiene (14), insbesondere unabhängig voneinander, verschiebbar sind.

8. Vorrichtung (10) nach Anspruch 6, **dadurch gekennzeichnet, dass** die erste, zweite, dritte und/oder vierte Patientenlagereinheit (40 bis 46) an der jeweiligen Schiene (12, 14) über eine manuell ohne weitere Hilfsmittel lös- und wiederherstellbare Befestigungseinheit (52) befestigt sind.

9. Vorrichtung (10) nach einem der Ansprüche 6 bis 8, **dadurch gekennzeichnet, dass** die erste Patientenlagereinheit (40) und die zweite Patientenlagereinheit (42) zur Lagerung des Oberkörpers des Patienten und/oder die dritte Patientenlagereinheit (44) und die vierte Patientenlagereinheit (46) zur Lagerung der Hüfte des Patienten ausgebildet sind.

10. Vorrichtung (10) nach einem der Ansprüche 6 bis 9, **dadurch gekennzeichnet, dass** die erste, zweite, dritte und/oder vierte Patientenlagereinheit (40 bis 46) jeweils eine Befestigungseinheit (52) zur Befestigung an der jeweiligen Schiene (12, 14), ein Auflagepad (50) zur Lagerung des Patienten und eine Höhenverstelleinheit (54), die zwischen der Befestigungseinheit (52) und dem Auflagepad (50) angeordnet ist und mit deren Hilfe der Abstand zwischen dem Auflagepad (50) und der Befestigungseinheit (52) einstellbar ist, umfasst.

11. Vorrichtung (10) nach Anspruch 10, **dadurch gekennzeichnet, dass** die Breite der Befestigungseinheit (52) und der Höhenverstelleinheit (54) jeweils in etwa der Breite der ersten bzw. zweiten Schiene (12, 14) entspricht.

12. Vorrichtung (10) nach Anspruch 10 oder 11, **dadurch gekennzeichnet, dass** die Auflagepads (50) einen bei einer gegenüberliegenden Anordnung der Auflagepads dem gegenüberliegenden Pad zugewandten Teilbereich mit einer homogenen Dicke und/oder mit einem röntgenstrahlendurchlässigen Material haben.

13. Vorrichtung (10) nach einem der Ansprüche 6 bis 12, **dadurch gekennzeichnet, dass** die erste Schiene (12), die zweite Schiene (14), die erste Patientenlagereinheit (40), die zweite Patientenlagereinheit (42), die dritte Patientenlagereinheit (44) und/oder die vierte Patientenlagereinheit (46) metallfrei, insbesondere aus kohlefaserverstärktem Kunststoff, ausgebildet sind.

## Claims

1. A device for supporting a patient to be X-rayed during an operation,
with a first rail (12) which can be fastened to an operating table,
with a second rail (14) which can be fastened to the operating table,
with at least one first connection unit (30) which connects the first end (16) of the first rail (12), which faces away from the operating table, to the first end (18) of the second rail (14), which faces away from the operating table, so that the two rails (12, 14) are arranged at a predetermined distance from one another,
with a first patient support unit (40) arranged on the first rail (12), for supporting a body area of the patient, and
with a second patient support unit (42) arranged on the second rail (14), for supporting a body area of the patient,
wherein the first patient support unit (40) is mounted exclusively on the first rail (12), and the second patient support unit (42) is mounted exclusively on the second rail (14), and said units are designed in such a manner that a material-free X-ray area (70) is formed between the two patient support units (40, 42), even in an opposite arrangement of the patient support units,
**characterised in that** the first patient support unit and the second patient support unit (40, 42) each comprise a fastening unit which comprises a U-shaped base body which can be placed from above onto the rectangular rails (12, 14), and that, at the end of one of the two flanks of the U-shaped base body, a latch (60) is swivelably arranged, which can be screwed via a screw (60) or nut on the end of the other flank of the U-shaped base body by means of a screw (60) which can be actuated manually without other auxiliary means.

2. The device (10) according to Claim 1, **characterised in that** the second end (22) of the first rail (12), which is opposite from the first end (16), and the second end (24) of the second rail (14), which is opposite from the first end (18), are connected to one another via a second connection unit (32).

3. The device (10) according to Claim 1, **characterised in that** the first rail (12) and the second rail (14) are connected exclusively via the first connection unit (30).

4. The device (10) according to any one of the preceding claims, **characterised in that** the first connection unit (30) is supported by a stand (20), in particular a height-adjustable stand (20).

5. The device (10) according to any one of the preceding claims, **characterised in that**, on the second end (22) of the first rail (12), which is opposite from the first end (16), a first fastening unit (26) is arranged for the fastening of the device (10) to the operating table, and, on the second end (24) of the second rail (14), which is opposite from the first end (18), a second fastening unit (28) is arranged for fastening the device (10) to the operating table.

6. The device (10) according to any one of the preceding claims, **characterised in that**, on the first rail (12), a third patient support unit (44) for supporting the patient is arranged, and/or, on the second rail (14), a fourth patient support unit (46) for supporting the patient is arranged.

7. The device (10) according to Claim 6, **characterised in that** the first patient support unit (40) and/or the third patient support unit (44) is/are slidable on the first rail (12), and/or the second patient support unit (42) and/or the fourth patient support unit (46) is/are slidable on the second rail (14), in particular independently of one another.

8. The device (10) according to Claim 6, **characterised in that** the first, second, third and/or fourth patient unit (40 to 46) is/are fastened to the respective rail (12, 14) via a fastening unit (52) that can be released and refastened manually without other auxiliary means.

9. The device (10) according to any one of Claims 6 to 8, **characterised in that** the first patient support unit (40) and the second patient support unit (42) are designed for supporting the upper body of the patient, and/or the third patient support unit (44) and the fourth patient support unit (46) are designed for supporting the hip of the patient.

10. The device (10) according to any one of Claims 6 to 9, **characterised in that** the first, second, third and/or fourth patient support unit (40 to 46) in each case comprise(s) a fastening unit (52) for fastening to the respective rail (12, 14), a support pad (50) for supporting the patient, and a height adjustment unit (54) which is arranged between the fastening unit (52) and the support pad (50) and by means of which the distance between the support pad (50) and the fastening unit (52) can be set.

11. The device (10) according to Claim 10, **characterised in that** the width of the fastening unit (52) and of the height adjustment unit (54) in each case corresponds approximately to the width of the first or second rail (12, 14).

12. The device (10) according to Claim 10 or 11, **characterised in that**, in an opposite arrangement of the support pads, the support pads (50) have a subsection facing the opposite pad, which has a homogeneous thickness and/or comprises an X-ray permeable material.

13. The device (10) according to any one of Claims 6 to 12, **characterised in that** the first rail (12), the second rail (14), the first patient support unit (40), the second patient support unit (42), the third patient support unit (44) and/or the fourth patient support unit (46) is/are designed to be metal-free, in particular made of carbon fibre-reinforced plastic.

## Revendications

1. Dispositif pour positionner un patient à radiographier pendant une opération, comportant un premier rail (12) pouvant être fixé à une table d'opération,
un deuxième rail (14) pouvant être fixé à la table d'opération, au moins une première unité de liaison (30) qui relie la première extrémité (16), opposée à la table d'opération, du premier rail (12) à la première extrémité (18), opposée à la table d'opération, du deuxième rail (14) de sorte que les deux rails (12, 14) sont disposés à un intervalle prédéterminé l'un par rapport à l'autre, une première unité de positionnement de patient (40) disposée sur le premier rail (12) pour le placement d'une zone corporelle du patient, et
une deuxième unité de positionnement de patient (42) disposée sur le deuxième rail (14) pour le placement d'une zone corporelle du patient,
dans lequel la première unité de positionnement de patient (40) est positionnée exclusivement sur le premier rail (12) et la deuxième unité de positionnement de patient (42) est positionnée exclusivement sur le deuxième rail (14) et est conçue de telle sorte que même dans le cas d'une disposition opposée des unités de positionnement du patient entre les deux unités de positionnement de patient (40, 42), une zone radiographique sans matériau (70) est conçue,
**caractérisé en ce que** la première et la deuxième unité de positionnement de patient (40, 42) présentent respectivement une unité de fixation qui comprend un corps de base en forme de U qui peut être monté par le haut sur les rails rectangulaires (12, 14) et **en ce qu'**à l'extrémité de l'un des deux flancs du corps de base en forme de U, un loquet (60) est disposé de façon à pouvoir basculer, qui peut être vissé par une vis (60) à l'extrémité de l'autre flanc du corps de base en forme de U à l'aide d'une vis (60) ou d'un écrou pouvant être actionné(e) manuellement sans autre moyen.

2. Dispositif (10) selon la revendication 1, **caractérisé en ce que** la deuxième extrémité (22) opposée à la première extrémité (16) du premier rail (12) et la deuxième extrémité (24) opposée à la première extrémité (18) du deuxième rail (14) sont reliées l'une à l'autre par une deuxième unité de liaison (32).

3. Dispositif (10) selon la revendication 1, **caractérisé en ce que** le premier rail (12) et le deuxième rail (14) sont reliés exclusivement par la première unité de liaison (30).

4. Dispositif (10) selon l'une quelconque des revendications précédentes, **caractérisé en ce que** la première unité de liaison (30) est soutenue par un montant (20) en particulier un montant (20) pouvant être réglé en hauteur.

5. Dispositif (10) selon l'une quelconque des revendications précédentes, **caractérisé en ce que** sur la deuxième extrémité (22) opposée à la première extrémité (16) du premier rail (12), une première unité de fixation (26) pour la fixation du dispositif (10) est fixée sur la table d'opération et sur la deuxième extrémité (24) opposée à la première extrémité (18) du deuxième rail (14), une deuxième unité de fixation (28) pour la fixation du dispositif (10) est fixée sur la table d'opération.

6. Dispositif (10) selon l'une quelconque des revendications précédentes, **caractérisé en ce que** sur le premier rail (12) est disposée une troisième unité de positionnement de patient (44) pour le positionnement du patient et/ou sur le deuxième rail (14) est disposée une quatrième unité de positionnement de patient (46) pour le positionnement du patient.

7. Dispositif (10) selon la revendication 6, **caractérisé en ce que** la première unité de positionnement de patient (40) et/ou la troisième unité de positionnement de patient (44) peuvent coulisser sur le premier rail (12) et/ou la deuxième unité de positionnement de patient (42) et/ou la quatrième unité de positionnement de patient (46) peuvent coulisser sur le deuxième rail (14), notamment indépendamment l'une de l'autre.

8. Dispositif (10) selon la revendication 6, **caractérisé en ce que** la première, la deuxième, la troisième et/ou la quatrième unité de positionnement de patient (40 à 46) sont fixées sur le rail respectif (12, 14) par une unité de fixation (52) pouvant être désengagée et réengagée de nouveau manuellement sans autre moyen.

9. Dispositif (10) selon l'une des revendications 6 à 8, **caractérisé en ce que** la première unité de positionnement de patient (40) et la deuxième unité de positionnement de patient (42) sont conçues pour le positionnement du haut du corps du patient et/ou la troisième unité de positionnement de patient (44) et la quatrième unité de positionnement de patient (46) sont conçues pour le positionnement des hanches du patient.

10. Dispositif (10) selon l'une des revendications 6 à 9, **caractérisé en ce que** la première, la deuxième, la troisième et/ou la quatrième unité de positionnement de patient (40 à 46) comprennent respectivement une unité de fixation (52) pour la fixation sur le rail respectif (12, 14), un tampon d'appui (50) pour le positionnement du patient et une unité de réglage de la hauteur (54) qui est disposée entre l'unité de fixation (52) et le tampon d'appui (50) et avec l'aide desquels l'intervalle entre le tampon d'appui (50) et l'unité de fixation (52) peut être réglé.

11. Dispositif (10) selon la revendication 10, **caractérisé en ce que** la largeur de l'unité de fixation (52) et l'unité de réglage de la hauteur (54) correspondent respectivement à peu près à la largeur du premier ou du deuxième rail (12, 14).

12. Dispositif (10) selon la revendication 10 ou 11, **caractérisé en ce que** les tampons d'appui (50), dans une disposition opposée au tampon d'appui, ont une zone partielle tournée vers le tampon opposé et présentant une épaisseur homogène et/ou un matériau transparent aux rayons X.

13. Dispositif (10) selon l'une des revendications 6 à 12, **caractérisé en ce que** le premier rail (12), le deuxième rail (14), la première unité de positionnement de patient (40), la deuxième unité de positionnement de patient (42), la troisième unité de positionnement de patient (44) et/ou la quatrième unité de positionnement de patient (46) sont exempts de métal, notamment sont conçus en une matière plastique renforcée en fibres de carbone.
